# EUROPÄISCHE PATENTSCHRIFT

(11) **EP 0 729 971 B1**
(45) Veröffentlichungstag und Bekanntmachung des Hinweises auf die Patenterteilung: **06.09.2000**
(21) Anmeldenummer: 96102394.2
(22) Anmeldetag: 17.02.1996
(51) Int. Cl.: C07H 17/08

(54) **Verfahren zur Herstellung von Ivermectin**
Method for the preparation of Ivermectin
Procédé de préparation d'Ivermectin

(30) Priorität: 01.03.1995 DE 19507018
(43) Veröffentlichungstag der Anmeldung: 04.09.1996
(73) Patentinhaber: BAYER AG, 51368 Leverkusen (DE)
(72) Erfinder: Arlt, Dieter, Prof. Dr., D-51065 Köln (DE); Bonse, Gerhard, Dr., D-51061 Köln (DE); Reisewitz, Friedhelm, Dr., D-42113 Wuppertal (DE)

(56) Entgegenhaltungen:
- EP-A- 0 001 689
- EP-A- 0 187 436
- EP-A- 0 266 131
- EP-A- 0 411 854

## Beschreibung

Ivermectin ist eine bekannte Verbindung, die hervorragende biologische Wirkungen besitzt und als Anthelmintikum, Ektoparasitizid, Insektizid und Akarizid breite Verwendung findet.

Es ist bekannt (EP 0 001 689), Ivermectin durch selektive katalytische Hydrierung aus Avermectin B₁ₐ und B_{1b} herzustellen. Avermectin wird biotechnologisch mit Hilfe von Streptomyces avermitilis gewonnen. Es besitzt fünf Doppelbindungen. Um Ivermectin aus diesem Ausgangsmaterial herzustellen, benötigt man einen selektiv wirkenden Katalysator, der nur die 22,23-Doppelbindung hydriert. 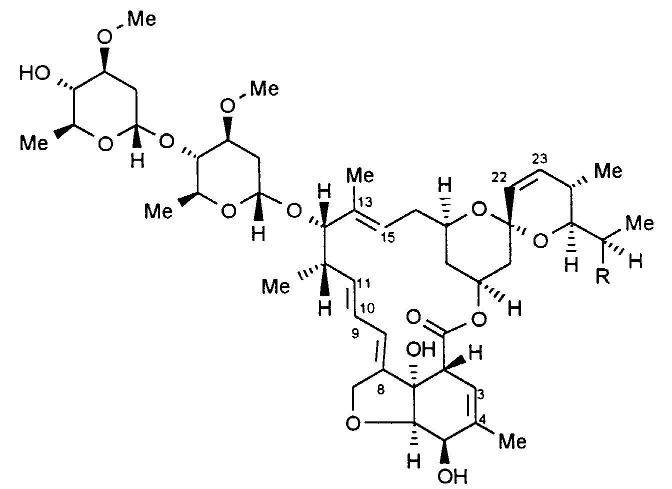 Avermectin B₁ₐ (R: -Ethyl)
Avermectin B_{1b} (R: -Methyl)

Es ist aus der EP 0 001 689 bekannt, Katalysatoren der allgemeinen Formel [(R)₃P]₃RhX für diesen Zweck verwenden zu können, bevorzugt wird der Wilkinson Katalysator [Ph₃P]₃RhCl eingesetzt. Der EP 0 001 689 ist zu entnehmen, daß relativ große Mengen dieses Katalysators (0,05 bis 0,5 mol/mol Avermectin) verwendet werden müssen, um die gewünschte Hydrierung zu erreichen.

Wegen des hohen Preises des Katalysators, insbesondere durch den Gehalt an teurem Rhodium bedingt, wurde deshalb vorgeschlagen (EP 0 059 616), zur Rückgewinnung der erheblichen Mengen dieses Edelmetalls die zur Herstellung von Ivermectin verwendet werden müssen, vorteilhaft einen besonderen Wiedergewinnungsprozeß einzusetzen. Die entsprechenden Beispiele der EP 0 059 616 zeigen, daß auch nach jahrelanger Prozeßentwicklung offenbar immer noch Mengen bis zu 10 % Katalysator bezogen auf Ausgangsmaterial Anwendung finden. Das in dieser Hinsicht günstigste Beispiel dieser Patentschrift gibt einen Katalysatoreinsatz von >1 % bezogen auf Edukt an.

Es besteht demnach ein Bedarf, Verfahren zu finden, die es erlauben, die Katalysatormenge, insbesondere die Menge an teurem Rhodium bei der Herstellung von Ivermectin aus Avermectin B₁ₐ und B_{1b} durch katalytische Hydrierung gegenüber dem bekannten Stand der Technik deutlich zu vermindern.

Überraschenderweise wurde nun gefunden, daß bei der Verwendung von Rh-Katalysatoren, die neben Phosphinen als Liganden auch Hydrazine enthalten, die notwendige Aufwandmenge für die selektive Hydrierung von Avermectin B₁ₐ und B_{1b} erheblich reduziert werden kann.

Gegenstand der vorliegenden Erfindung ist demnach ein Verfahren zur Herstellung von Ivermectin aus Gemischen von Avermectin B₁ₐ und B_{1b} durch selektive katalytische Hydrierung der 22,23-Doppelbindung im Ausgangsmaterial, dadurch gekennzeichnet, daß als Katalysator eine Rh-Komplexverbindung eingesetzt wird, die durch die Umsetzung eines Rhodiumsalzes, eines Hydrazins oder Hydrazinsalzes und eines tertiären Phosphins oder eines Rhodium-Phosphin-Komplexes mit einem Hydrazin oder einem Hydrazin-Salz erhalten wird.

Katalysatoren dieser Art und ihre Herstellung sind bekannt; siehe z.B. EP 0 086 046, EP 0 283 615 und Tetrahedron Vol. 7, No. 19/20, S. 2087-2089 (1988).

Das neue Verfahren zeichnet sich dadurch aus, daß im Unterschied zum bekannten Stand der Technik Katalysatormengen von <1 % bezogen auf die eingesetzte Menge des Ausgangsmaterials und in einer Rh-Konzentration, die nur ≤1/10 der nach dem Stand der Technik erforderlichen Konzentration beträgt, wirkungsvoll mit hoher Selektivität zum gewünschten Produkt führen.

Die katalytische Hydrierung des erfindungsgemäßen Verfahrens wird in üblichen Lösungsmitteln, wie z.B. Alkoholen oder aromatischen Kohlenwasserstoffen gegebenenfalls unter Zusatz von aliphatischen Kohlenwasserstoffen ausgeführt, beispielsweise in Methanol, Methanol-Cyclohexan-Gemischen oder Toluol, das bevorzugt eingesetzt wird. Die Temperatur während der Umsetzung liegt etwa im Bereich von 60 bis 100°C, der Wasserstoffdruck im Bereich von etwa 1 bis 150 bar. Um die Reaktionszeit zu verkürzen, ist es zweckmäßig, bei Überdruck zu arbeiten, bevorzugt wird ein Bereich von 20 bis 100 bar.

Die Aufarbeitung des Reaktionsgemisches nach der Hydrierung erfolgt in an sich bekannter Weise, z.B. nach dem in der EP 0 059 616 beschriebenen Verfahren oder alternativ durch Adsorption des Rhodiums an einem Reaktionsharz und anschließende Aufreinigung des Rh-freien produktes z.B. durch bekannte Kristallisationsverfahren.

### Beispiel 1

A) Katalysator (bekannt):
   Rhodiumtrichlorid-trihydrat (1,00 g; 3,80 mmol) wurde unter Erwärmung (70°C) in Wasser (5,0 ml) gelöst. Unter Stickstoffatmosphäre wurde dann innerhalb von 20 Minuten eine Lösung von Triphenylphosphin (1,95 g; 7,43 mmol) in Aceton (25,0 ml) hinzugegeben. Nach 10 Minuten wurde unter Rühren Hydrazinhydrat (1,90 ml; 39,09 mmol) hinzugefügt und die Mischung für 3 Stunden auf Rückflußtemperatur erwärmt, anschließend noch 1 Stunde auf 45°C gehalten. Der dann ausgefallene kristalline Feststoff wurde unter Stickstoff abfiltriert, mit wenig Aceton und anschließend mit Diethylether gewaschen. Man erhielt 1,05 g eines orangefarbenen Feststoffes.
B) Hydrierung (neu):
   Der nach A) erhaltene Katalysator (10 mg) wurde in Toluol (25 ml) gelöst und unter Argon zu der Lösung einer Mischung (1,1 g) aus Avermectin B₁ₐ (96 %) und Avermectin B_{1b} (4 %) und von 100 mg Triphenylphosphin in Toluol (25 ml) in einem V4A-Autoklaven gegeben. Dieses Edukt wurde dann bei 88°C unter einem Wasserstoffdruck von 20 bar unter Rühren der Lösung hydriert. Nach 10 Stunden ergab eine HPLC-Analyse einen Gehalt von 86 % Dihydro-avermectin B₁ₐ und von 4 % Dihydro-avermectin B_{1b}, sowie von 3 % Tetrahydro-avermectin B₁ₐ.

### Beispiel 2

Der nach Beispiel 1A) erhaltene Katalysator (15 mg) wurde in einer Lösung von 8,6 g Avermectin B₁ₐ und B_{1b} (Gehalt 77,5 % bzw. 18,0 %) in einer Mischung von 33,3 ml Methanol und 16,7 ml Cyclohexan gelöst und nach Zusatz von 80 mg Triphenylphosphin unter intensiver Vermischung 8 Stunden bei einer Temperatur von 88°C und einem Wasserstoffdruck von 20 bar hydriert. Das erhaltene Produkt enthielt neben 6,2 % Edukt 84 % 22,23-Dihydro-avermectinen.

### Beispiel 3

A) in situ-Bereitung des Katalysators:
   Eine Mischung von 4,45 mg Rhodiumtrichlorid-trihydrat, 11,33 mg Triphenylphosphin, 22,5 µl Wasser und 3,0 ml Aceton wurden 30 Minuten auf 60°C erwärmt, anschließend 10 µl Hydrazinhydrat und weitere 3,0 ml Aceton zugegeben. Unter Rühren und Rückflußkühlung wurde dieses Gemisch weitere 20 Stunden auf 60°C erwärmt.
B) Die nach 3A) erhaltene Mischung wurde zu einer Lösung von 8,6 g Avermectin B₁ₐ und B_{1b} in 50 ml Toluol gegeben. Die Mischung wurde 10 Stunden bei einer Temperatur von 87°C und einem Wasserstoffdruck von 10 bar hydriert. Man erhielt ein Produkt mit einem Gehalt von 92,3 % 22,23-Dihydro-avermectinen und 3,6 % Tetrahydro-avermectinen (nach HPLC-Analyse).

## Patentansprüche

1. Verfahren zur Herstellung von 22,23-Dihydro-avermectinen durch selektive katalytische Hydrierung von Avermectin B₁ₐ und/oder B_{1b}, dadurch gekennzeichnet, daß als Katalysator eine Rh-Komplexverbindung verwendet wird, die durch die Umsetzung des Rhodiumsalzes, eines Hydrazins oder eines Hydrazinsalzes und eines tertiären Phosphins oder einer Rh-Phosphin-Komplexverbindung mit einem Hydrazin oder einem Hydrazinsalz erhalten wird.

2. Verfahren nach Anspruch 1, dadurch gekennzeichnet, daß die katalytische Hydrierung in einem Temperaturbereich von 60 bis 100°C und einem H₂-Druck von 1 bis 150 bar durchgeführt wird.

3. Verfahren nach Anspruch 1, dadurch gekennzeichnet, daß die katalytische Hydrierung in aromatischen Kohlenwasserstoffen als Lösungsmittel durchgeführt wird.

## Claims

1. Process for the preparation of 22,23-dihydroavermectins by selective catalytic hydrogenation of avermectin B₁ₐ and/or B_{1b}, characterized in that, as catalyst, an Rh complex compound which is obtained by the reaction of the rhodium salt, a hydrazine or a hydrazine salt and a tertiary phosphine or an Rh-phosphine complex compound with a hydrazine or a hydrazine salt is used.

2. Process according to Claim 1, characterized in that the catalytic hydrogenation is carried out in a temperature range from 60 to 100°C and an H₂ pressure from 1 to 150 bar.

3. Process according to Claim 1, characterized in that the catalytic hydrogenation is carried out in aromatic hydrocarbons as solvents.

## Revendications

1. Procédé de préparation de 22,23-dihydroavermectines par hydrogénation catalytique sélective d'Avermectines B₁ₐ et/ou B_{1b}, caractérisé en ce que l'on met en oeuvre comme catalyseur, un composé complexe du Rh, qui est obtenu par réaction d'un sel de rhodium, d'une hydrazine ou d'un sel d'hydrazine et d'une phosphine tertiaire, ou d'un composé complexe Rh-phosphine avec une hydrazine ou un sel d'hydrazine.

2. Procédé suivant la revendication 1, caractérisé en ce que l'hydrogénation catalytique est réalisée dans un intervalle de température allant de 60 à 100°C et à une pression de H₂ allant de 1 à 150 bar.

3. Procédé suivant la revendication 1, caractérisé en ce que l'hydrogénation catalytique est réalisée dans un hydrocarbure aromatique comme solvant.
